# EUROPEAN PATENT APPLICATION

(11) **EP 4 446 313 A1**
(43) Date of publication of application: **16.10.2024**
(21) Application number: 22904248.6
(22) Date of filing: 07.12.2022
(51) Int. Cl.: C07D 233/90, A61K 31/4164, A61P 17/00, A61P 31/04, A61P 31/10

(54) **HYDRATE CRYSTAL OF 5-CHLORO-4-(3-CHLORO-4-METHYLPHENYL)-1H-IMIDAZOLE-2-CARBONITRILE**

(30) Priority: 08.12.2021 JP 2021199461
(71) Applicant: Ishihara Sangyo Kaisha, Ltd., Osaka-shi, Osaka 550-0002 (JP)
(72) Inventor: SHIKAMA Hiroshi, Osaka-shi, Osaka 550-0002 (JP); HIGUCHI Koji, Osaka-shi, Osaka 550-0002 (JP); ATSUMI Shogo, Osaka-shi, Osaka 550-0002 (JP); SHIOTA Hiroto, Osaka-shi, Osaka 550-0002 (JP); IMURA Takayuki, Osaka-shi, Osaka 550-0002 (JP)
(74) Representative: Regimbeau
(86) International application number: PCT/JP2022/045047
(87) International publication number: WO 2023/106320

(57) **Abstract**

The present invention relates to a hydrate crystal of 5-chloro-4-(3-chloro-4-methylphenyl)-1H-imidazole-2-carbonitrile.

## Description

### Cross-Reference to the Related Applications

The present patent application involves a claim of priority to Japanese Patent Application No. 2021-199461 filed on December 8, 2021, the entire disclosure content of which is regarded as part of the present specification by reference.

### Technical Field

The present invention relates to a hydrate crystal of 5-chloro-4-(3-chloro-4-methylphenyl)-1H-imidazole-2-carbonitrile.

### Background Art

Patent Literature 1 discloses 5-chloro-4-(3-chloro-4-methylphenyl)-1H-imidazole-2-carbonitrile. The literature relates to a technique regarding imidazole compounds and pest control agent containing the compounds. As an example of an intermediate for synthesizing imidazole compounds of interest, 5-chloro-4-(3-chloro-4-methylphenyl)-1H-imidazole-2-carbonitrile is described in Table 1 as the intermediate No. 89.

By the way, a fungus of the genus Malassezia is a basidiomycetous yeast that resides on the skin of humans and animals. The fungus of the genus Malassezia grows using lipid as the nutrient source, and therefore, it settles in an area with a lot of sebum, and is known to be the cause of skin diseases such as tinea versicolor, seborrheic dermatitis, folliculitis, atopic dermatitis, and psoriasis vulgaris.

Examples of the conventional therapeutic agent for skin diseases caused by the fungus of the genus Malassezia include selenium disulfide, zinc 2-mercaptopyridine N-oxide, piroctone olamine, imidazole compounds such as miconazole nitrate and ketoconazole, and triazole compounds such as itraconazole and fluconazole.

Usually, normal skin inhabitants have so-called barrier effects such as effects of preventing scalp odor due to the growth of microbes on skin and effects of maintaining a weakly acidic state by moderately decomposing sebum. The conventional therapeutic agents sterilize not only pathogenic microbe but also normal skin inhabitants, which raises concerns that the barrier effects on the skin will be destroyed. Also, selenium disulfide has been pointed out to have side effects such as oral toxicity, loss of hair, skin rash, debility, malaise, and hair discoloration, and zinc 2-mercaptopyridine N-oxide has been pointed out to have a sulfurous odor and to raise concerns about endocrine disrupting chemicals. Further, imidazole compounds such as miconazole nitrate and triazole compounds such as itraconazole have an action of inhibiting lipid synthesis in microbes. However, these compounds need to be administered for a long period of time, because it takes time to exhibit sufficient effects due to their nature of exhibiting antimicrobial activities by inhibiting the biosynthesis of cell membranes in microbes. Continuously administering the same agent over a long period of time is known to significantly increase the risk of developing resistant microbes.

As described above, lipid synthesis inhibitors do not exhibit antimicrobial activities immediately after the agent is administered. Consequently, the lipid synthesis inhibitors are not suitable for preventing or treating skin diseases by an administration method that requires an immediate effect of exhibiting antimicrobial activities, such as a medical shampoo. Hence, there has been a demand for developing compounds and therapeutic agents that are excellent in safety, have a mechanism of action different from that of conventional therapeutic agents, and immediately exhibit antimicrobial activities.

### Citation List

### Patent Literature

Patent Literature 1: Japanese Patent Laid-Open No. 1-131163

### Summary of Invention

The present disclosers have found that 5-chloro-4-(3-chloro-4-methylphenyl)-1H-imidazole-2-carbonitrile can be used as a novel antimicrobial agent for a non-human animal or an antifungal agent for a human.

However, the present disclosers have further studied and found that 5-chloro-4-(3-chloro-4-methylphenyl)-1H-imidazole-2-carbonitrile may easily absorb moisture at room temperature. Hygroscopic substances are usually handled with great care to avoid moisture absorption. When a hygroscopic substance is an active pharmaceutical ingredient, the handling conditions are restricted, such as a storage container, a storage location, an expiry date, and a dividing bag for a standard product for testing. Accordingly, high stability to changes in temperature and humidity is required.

In addition, the present disclosers have studied and found that 5-chloro-4-(3-chloro-4-methylphenyl)-1H-imidazole-2-carbonitrile has strong electrification properties, and its adhesion to a production facility such as piping, experimental equipment, or packaging materials may strongly occur. Accordingly, there are concerns about processes, quality or, the like, such as a decrease in yield due to the adhesion to a production facility such as piping or packaging materials, deterioration of mixing uniformity, and the cause of weighing errors.

The present disclosers have further studied and found out this time that, by making 5-chloro-4-(3-chloro-4-methylphenyl)-1H-imidazole-2-carbonitrile into hydrate crystals, they can be advantageously used in immediately exerting excellent antimicrobial activities on fungi, including the fungus of the genus Malassezia. In addition, the present disclosers have found out that the above-described hydrate crystals can be advantageously used in immediately exerting excellent antimicrobial activities on bacteria, including a bacterium of the genus Staphylococcus. Further, the present disclosers have found out that the above-described hydrate crystals are not susceptible to temperature or humidity in the environment, and are excellent in stability. The present disclosers have also found out that making 5-chloro-4-(3-chloro-4-methylphenyl)-1H-imidazole-2-carbonitrile into hydrate crystals can cause triboelectrification properties to be lowered, and the concerns about processes, quality, or the like due to the electrification such as the adhesion to a facility or the like to be weakened. The present disclosure is based on these findings.

Therefore, according to an embodiment of the present disclosure, provided is a hydrate crystal of 5-chloro-4-(3-chloro-4-methylphenyl)-1H-imidazole-2-carbonitrile.

The hydrate crystal of 5-chloro-4-(3-chloro-4-methylphenyl)-1H-imidazole-2-carbonitrile (hereinafter, also simply referred to as "hydrate crystal") of the present disclosure can take effect of killing fungi such as the fungus of the genus Malassezia or bacteria such as the bacterium of the genus Staphylococcus, or suppressing the growth thereof, in the same manner as anhydrous crystals. In other words, the hydrate crystal of the present disclosure can be expected to be used for preventing, ameliorating or treating skin diseases caused by fungi or bacteria, such as tinea versicolor, folliculitis, seborrheic dermatitis, atopic dermatitis, and psoriasis vulgaris. Further, the hydrate crystal of the present disclosure is in a stable state with almost no change in moisture content, regardless of a high humidity or dry environment, and therefore, it is advantageous in producing a preparation in a stable manner. Moreover, the hydrate crystal of the present disclosure has low triboelectrification properties and fast attenuation speed of the amount of electrification, and the concerns about processes, quality, or the like, due to the electrification such as the adhesion to a facility or the like can be weakened, and therefore, it can be advantageously used in efficiently producing a preparation.

### Brief Description of Drawings

[Figure 1] Figure 1 shows a powder X-ray diffraction (XRD) pattern of the hydrate crystal of 5-chloro-4-(3-chloro-4-methylphenyl)-1H-imidazole-2-carbonitrile obtained in Production Example 1.
[Figure 2] Figure 2 shows a powder X-ray diffraction pattern of the hydrate crystal of 5-chloro-4-(3-chloro-4-methylphenyl)-1H-imidazole-2-carbonitrile obtained in Production Example 2.
[Figure 3] Figure 3 shows a powder X-ray diffraction pattern of the anhydrous crystal of 5-chloro-4-(3-chloro-4-methylphenyl)-1H-imidazole-2-carbonitrile used as a raw material.
[Figure 4] Figure 4 is a comparison graph of XRD charts for a water-added anhydrate crystal, an anhydrate crystal, and hydrate crystals (Production Examples 1 and 2).
[Figure 5A] Figure 5Ais a thermogravimetric-differential thermal analysis (TG-DTA) chart of the anhydrate crystal.
[Figure 5B] Figure 5B is a thermogravimetric-differential thermal analysis (TG-DTA) chart of the water-added anhydrate crystal.
[Figure 5C] Figure 5C is a TG-DTA chart of the hydrate crystal (Production Example 1).
[Figure 5D] Figure 5D is a TG-DTA chart of the hydrate crystal (Production Example 2).
[Figure 6A] Figure 6Ais photographs of the anhydrate crystal, the hydrate crystal (Production Examples 1) and the hydrate crystal (Production Examples 2) taken at 500x magnification with a scanning electron microscope.
[Figure 6B] Figure 6B is photographs of the anhydrate crystal, the hydrate crystal (Production Examples 1) and the hydrate crystal (Production Examples 2) taken at 100x magnification with a scanning electron microscope.
[Figure 7A] Figure 7Ais an infrared absorption spectrum of the anhydrate crystal.
[Figure 7B] Figure 7B is an infrared absorption spectrum of the hydrate crystal (Production Examples 2).
[Figure 8] Figure 8 is a comparison graph of Raman spectroscopy spectra of an anhydrate crystal-1, an anhydrate crystal-2, the hydrate crystal (Production Examples 1) and the hydrate crystal (Production Examples 2).

### Description of Embodiments

Hereinafter, the present invention will be described by way of a preferred embodiment. However, the following embodiments are merely examples for illustrating the present invention, and the present invention is not limited thereto.

The hydrate crystal of the present disclosure substantially consists of a hydrate crystal of 5-chloro-4-(3-chloro-4-methylphenyl)-1H-imidazole-2-carbonitrile. The hydrate crystal of the present disclosure is preferably a monohydrate crystal. 5-chloro-4-(3-chloro-4-methylphenyl)-1H-imidazole-2-carbonitrile is a compound described as the intermediate No. 89 in Table 1 of Patent Literature 1.

The term "hydrate crystal" used herein encompasses crystalline polymorphs of hydrate crystals. In addition, the phrase "substantially consists of a hydrate crystal" means that the content of residues other than the hydrate crystal in the hydrate crystal is less than 20% (by mass). The content of the residues in the hydrate crystal is preferably 10% (by mass) or less, more preferably 5% (by mass) or less, further preferably 1% (by mass) or less, and further more preferably 0.5% (by mass) or less.

Examples of the residues include production raw materials, crystalline polymorphs other than the hydrate crystal, water of adhesion, and a mixture thereof. A specific example of the residues includes an anhydrous crystal of 5-chloro-4-(3-chloro-4-methylphenyl)-1H-imidazole-2-carbonitrile.

### (Powder X-Ray Diffraction Pattern)

According to a preferred aspect of the present disclosure, the hydrate crystal can be characterized by a powder X-ray diffraction pattern. According to an aspect, at least one peak is present at any diffraction angles (20 ± 0.2°) of 9.8, 12.8, 14.6, 16.0, 19.5, 24.2, 25.3, 25.7, 26.7, 27.7, 28.5, 28.8, 29.5, 30.3, 32.3, 33.1, 34.1, 35.7, 36.4, 37.4, 39.5, 40.1, 43.3, or 44.3, in the powder X-ray diffraction pattern of the hydrate crystal. In addition, according to a preferred aspect, at least one peak is present at any diffraction angles (2θ ± 0.2°) of 9.8, 12.8, 14.6, 16.0, 19.5, 25.3, 25.7, 26.7, or 29.5, in the powder X-ray diffraction pattern of the hydrate crystal. Further, the at least one peak is preferably 5 or more, more preferably 7 or more, and still more preferably 9 or more peaks. Also, according to an embodiment of the present disclosure, peaks present at any of the above-mentioned diffraction angles are top peaks with highest intensity, that is, ones selected in descending order of peak intensity.

According to another aspect, the top 5 peaks with highest intensity are each present at any diffraction angles (2θ ± 0.2°) of 9.8, 12.8, 14.6, 16.0, 19.5, 24.2, 25.3, 25.7, 26.7, 27.7, 28.5, 28.8, 29.5, 30.3, 32.3, 33.1, 34.1, 35.7, 36.4, 37.4, 39.5, 40.1, 43.3, or 44.3, in the powder X-ray diffraction pattern of the hydrate crystal. In addition, according to another preferred aspect, the top 5 peaks with highest intensity are each present at any diffraction angles (2θ ± 0.2°) of 9.8, 12.8, 14.6, 16.0, 19.5, 25.3, 25.7, 26.7, or 29.5, in the powder X-ray diffraction pattern of the hydrate crystal. Preferably. the top 7 peaks with highest intensity are each present at any of the above diffraction angles.

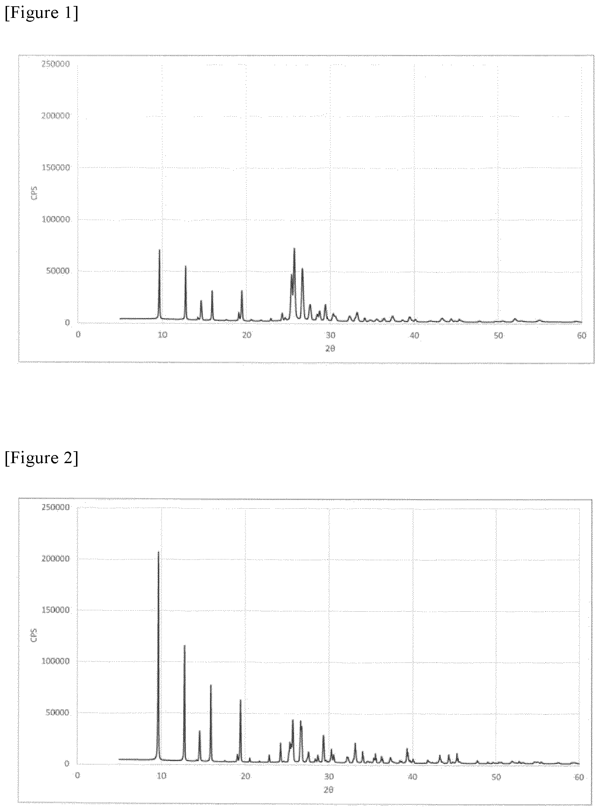

According to yet another aspect of the present disclosure, the hydrate crystal can be characterized by a powder X-ray diffraction pattern substantially identical to that in Figure 1. Here, the phrase "substantially identical" means that peak positions are identical except the range of measurement errors.

According to yet another aspect of the present disclosure, the hydrate crystal can be characterized by a powder X-ray diffraction pattern substantially identical to that in Figure 2. Here, the phrase "substantially identical" means that peak positions are identical except the range of measurement errors.

Here, a powder X-ray diffraction pattern of an anhydrous crystal has major peaks at positions of diffraction angles (2θ ± 0.2°) of 10.0, 10.7, 11.3, 15.6, 16.1, 20.1, 21.2, 22.3, 23.8, 26.8, 27.3, 28.6, 29.1, and 30.6. In the powder X-ray diffraction pattern of the anhydrous crystal, each of the top 5 peaks with highest intensity are often present at any diffraction angles (2θ ± 0.2°) of 10.7, 11.3, 16.1, 22.3, 23.8, or 26.8. Therefore, by comparing the top 5 peaks with highest intensity, anhydrous crystals and hydrate crystals can be clearly distinguished.

The powder X-ray diffraction patterns can be measured using a Cu-Kα radiation source, and more specifically, they can be obtained by measuring Cu-Kα as a radiation source at a tube voltage of 40 kV and a tube current of 40 mA. Note that the powder X-ray diffraction patterns can be measured using a known apparatus. However, when measured values differ depending on the apparatus, values obtained by measuring with a high-performance X-ray diffraction apparatus (for example, D8 DISCOVER, manufactured by Bruker AXS GmbH) can be used. Further details about the measurement of the powder X-ray diffraction patterns conform to Test Examples to be described later.

### (Infrared Absorption Spectrum)

According to an aspect of the present disclosure, an infrared absorption spectrum of the hydrate crystal measured by a potassium bromide disk method (see The Japanese Pharmacopoeia, 18th Edition, 2.25 "Infrared Spectrophotometry") has at least one peak at a position (cm-1) selected from 835 ± 2, 860 ± 2, 1014 ± 2, 1053 ± 2, 1157 ± 2, 1315 ±2, 1427 ± 2, 1454 ±2, 1500 ± 2, 1581 ± 2, 1654 ± 2 and 2245 ± 2. The at least one peak is preferably 5 or more, more preferably 7 or more, and still more preferably 9 or more peaks. Also, according to an embodiment of the present disclosure, peaks present at any of the above-mentioned diffraction angles are selected in descending order of peak intensity. Details of a technique of measuring an infrared absorption spectrum conform to Test Examples to be described later.

### (Raman Spectroscopy Spectrum)

According to an aspect of the present disclosure, a Raman spectroscopy spectrum (see The Japanese Pharmacopoeia, 18th Edition, 2.26 "Raman Spectroscopy") of the hydrate crystal has at least one peak at a position (cm-1) selected from 1021 ± 2, 1152 ± 2, 1240 ± 2, 1281 ± 2, 1379 ± 2, 1409 ± 2, 1424 ± 2, 1451 ± 2, 1507 ± 2, 1573 ± 2, 1607 ± 2 and 2241 ± 2. The at least one peak is preferably 5 or more, more preferably 7 or more, and still more preferably 9 or more peaks. Also, according to an embodiment of the present disclosure, peaks present at any of the above-mentioned diffraction angles are selected in descending order of peak intensity. Details of a technique of measuring a Raman spectroscopy spectrum conform to Test Examples to be described later.

### (Karl Fischer Moisture Content)

According to a preferred aspect of the present disclosure, the hydrate crystal can be characterized by a Karl Fischer moisture content. According to an aspect, the hydrate crystal has a moisture content of 6.5 to 6.8% by mass, and preferably 6.6 to 6.7% by mass, measured by a Karl Fischer method (volumetric titration method). Here, the molecular weight of 5-chloro-4-(3-chloro-4-methylphenyl)-1H-imidazole-2-carbonitrile is 252.10, and the molecular weight of water is 18.00. Accordingly, it can be calculated as n ≈ 1 from a calculation formula of moisture content (e.g., 6.6% by mass) ≈ 100 × (18.00 × n)/(252.10 + 18.00 × n). Hence, in consideration for the results of Test Examples to be described later, the hydrate crystal of the present disclosure is considered to be monohydrate, based on the value of moisture content.

The Karl Fischer moisture content in the present disclosure can be measured in an atmosphere of 25°C, in conformance with the method described in The Japanese Pharmacopoeia, 18th Edition, "2.48 Water Determination (Karl Fischer Method)", Volumetric titration. Further details about the measurement of the Karl Fischer moisture content conform to Test Examples to be described later.

### (Hydrate Crystal of First Form and Production Method Thereof)

The method for preparing the hydrate crystal of the present disclosure is not particularly limited. An example of the preparation method is a method in which an anhydrous crystal is placed in a stability test chamber with the temperature and humidity adjusted, and left to stand still until the crystal reaches to a saturated state with no more change in weight. Hereinafter, the hydrate crystal produced by the method is also referred to as hydrate crystal of a first form, and corresponds to Production Example 1 in Examples to be described later. Examples of conditions of temperature and relative humidity of a stability test chamber in the method include conditions of 40 to 80°C and 60 to 100%, and preferably 50 to 70°C and 70 to 90%, respectively. Examples of setlling time include 10 to 30 days, and preferably 15 to 25 days.

### (Hydrate Crystal of Second Form and Production Method Thereof)

Another example of the preparation method is a method in which an anhydrous crystal is dissolved in alcohol, the solution is added with water followed by being stirred and filtered under reduced pressure, and then dried. Hereinafter, the hydrate crystal produced by the method is also referred to as hydrate crystal of a second form, and corresponds to Production Example 2 in Examples to be described later. After filtration under reduced pressure, the filtered crystal may be washed with an alcohol aqueous solution. In this method, examples of alcohol include, but not limited to, lower alcohols, such as methanol, ethanol, and isopropyl alcohol. Water may be added all at once, or may be added dropwise over about an hour. Examples of stirring time include 6 to 24 hours, and preferably 10 to 20 hours. Examples of drying method include an air drying method at 10 to 60°C, relative humidity of 1 to 60% and for 6 to 48 hours, and preferably 20 to 40°C, relative humidity of 20 to 60% and for 10 to 20 hours.

Note that anhydrous crystals can be prepared by the methods described in Japanese Patent Laid-Open Nos. 1-131163, 8-283243 and 8-225539, or in conformance therewith.

### (Thermal Analysis)

According to an aspect, the hydrate crystal of the present disclosure has at least one endothermic peak, and preferably two within a range selected from 93 to 100°C and 135 to 145°C in thermogravimetric-differential thermal analysis (TG-DTA) or differential scanning calorimetry analysis (DSC). From the results of Examples to be described later, the above endothermic peaks are considered to be due to the desorption of added water from the hydrate crystal. The measurement methods of thermogravimetric-differential thermal analysis (TG-DTA) or differential scanning calorimetry analysis (DSC) can be performed in conformance with the description in The Japanese Pharmacopoeia, 18th Edition, and further details conform to Test Examples to be described later.

Also, as shown in Test Examples to be described later, it has been found out from the study of the present disclosers that endothermic peak patterns in the above thermal analysis differ depending on whether the hydrate crystal is the first form or the second form. Without wishing to be bound by theory, it is considered that differences in the growth degree and orientation of the crystal faces, and the like occur due to the difference in the production methods of the two, causing the endothermic peak patterns to be affected.

When the above hydrate crystal is the first form, the hydrate crystal of the first form exhibits one endothermic peak within the above temperature range in thermogravimetric-differential thermal analysis (TG-DTA) or differential scanning calorimetry analysis (DSC). The one endothermic peak of the hydrate crystal of the first form preferably falls in a range of 93 to 100°C, and more preferably in a range of 94 to 97°C.

When the above hydrate crystal is the second form, the hydrate crystal of the second form exhibits two endothermic peaks within the above temperature range in thermogravimetric-differential thermal analysis (TG-DTA) or differential scanning calorimetry analysis (DSC). The respective two endothermic peaks of the hydrate crystal of the second form preferably fall in a range of 95 to 99°C and 137 to 142°C, and more preferably in a range of 96 to 98°C and 138 to 141°C.

Note that, according to an aspect, the hydrate crystal exhibits one endothermic peak within the range of 165 to 170°C in thermogravimetric-differential thermal analysis (TG-DTA) or differential scanning calorimetry analysis (DSC), regardless of whether the hydrate crystal is the first form or the second form. Since this peak also occurs in an anhydrate crystal, it is considered to be due to the dissolution of a crystal of 5-chloro-4-(3-chloro-4-methylphenyl)-1H-imidazole-2-carbonitrile.

The hydrate crystal of the present disclosure is significantly low in electrification properties compared to anhydrate crystals, and can reduce the amount of electrification and maintain it at a low level, as shown in Examples to be described later. Such a hydrate crystal has a low adherence with easy handling compared to anhydrate crystals, and it can be particularly advantageously used in industrially producing preparations.

When holding the hydrate crystal in an environment of 25°C and 50% RH for 20 minutes, the electrification voltage of the hydrate crystal is, for example, -4.5 to -1 V, preferably -4.5 to -1 V, more preferably -4 to -1.5 V, and even more preferably -3.5 to -1.5 V

In addition, when holding the hydrate crystal in an environment of 25°C and 50% RH for 20 minutes, the decrease rate of the amount of electrification of the hydrate crystal is, for example, 5 to 40%, preferably 5 to 30%, more preferably 5 to 20%, and even more preferably 5 to 15%.

Note that a method for measuring the electrification voltage and the reduced rate of the amount of electrification of the hydrate crystal can be performed in conformance with the description of Test Example 5: Test for Triboelectrification Properties, which will be described later.

Also, the shape of the hydrate crystal may be a columnar, massive, substantially rectangular parallelepiped shape, or the like, but is preferably a columnar shape. According to an embodiment of the present disclosure, when the hydrate crystal is the second form, the shape of the hydrate crystal is a columnar shape. Without wishing to be bound by theory, in the production process of the second form, since the system is relatively homogeneous, crystals are considered to be stably generated with a regular columnar structure.

The hydrate crystal of the present disclosure can be used as an agent or formulation as it is. In addition, the hydrate crystal of the present disclosure can be combined with other pharmacologically acceptable additional ingredients such as carriers, active ingredients, pharmacological ingredients, or cosmetic ingredients, as necessary, to form a composition and may be formulated by a conventional method for use. Therefore, according to an aspect of the present disclosure, provided is a composition or preparation (hereinafter, also simply referred to as "preparation") including the hydrate crystal, and a pharmacologically acceptable additional ingredient, as desired.

According to an aspect, the preparation of the present disclosure can be used as an antimicrobial agent. As mentioned above, the antimicrobial agent of the present disclosure can be any agent as long as it contains a hydrate crystal, and can further contain other additional ingredients such as carriers, active ingredients, pharmacological ingredients, or cosmetic ingredients, as necessary.

According to an aspect, the preparation of the present disclosure has a content of the hydrate crystal of 0.00001 to 30% by mass, preferably 0.0001 to 10% by mass, and more preferably 0.001 to 0.05% by mass.

According to an aspect, the carrier used in the preparation is preferably a pharmaceutically or cosmetically acceptable carrier, and examples thereof include a carrier that can generally be pharmaceutically or cosmetically used, such as an excipient, a coating agent, a binder, a bulking agent, a disintegrant, a lubricant, a diluent, an osmotic regulator, a pH adjuster, a dispersant, an emulsifier, a preservative, a stabilizer, an antioxidant, a colorant, a UV absorber, a humectant, a thickener, an activity enhancer, a fragrance, a flavorant, and an odor improving agent.

In addition, examples of the other additional ingredients include a humectant, an antiinflammatory agent, a bactericide, a antimicrobial agent, a UV protectant, a cell activator, and a makeup ingredient.

The preparation containing the hydrate crystal of the present disclosure can be provided as a pharmaceutical, a quasi-drug, a veterinary drug, or a cosmetic. Among others, it is preferably used as a pharmaceutical and a veterinary drug.

The preparation may be orally or parenterally administered. Examples of the dosage form for oral administration include solid administration forms such as dusting powders, granules, water dispersible granules, wettable powders, tablets, coated tablets, fine granules, powders, pills, and capsules (including a form that envelops with a water-soluble film), and liquid administration forms such as elixirs, syrup, aqueous suspending agents, oily suspending agents, microemulsion preparations, suspoemulsion preparations, water solutions, emulsions, liquids, and paste. Examples of the dosage form for parenteral administration include injections, infusions, topical, external preparations, transdermal, transmucosal, transnasal, enteral, inhalation, suppositories, bolus, and patches. Since the hydrate crystal of the present disclosure is solid, it is necessary to pass through a solid; however, the hydrate crystal of the present disclosure has low triboelectrification properties and fast attenuation speed of the amount of electrification, and therefore, the concerns about processes, quality or the like, due to the electrification such as the deterioration of mixing uniformity and the adhesion to a facility or the like can also be alleviated for any dosage forms of preparations. It is preferable that the dosage form of the preparation be, among these, dusting powders or tablets, which are often used as solids in the process, because significant effects can be obtained.

According to an aspect, examples of the form of the preparation of the present disclosure include any forms that can be used in cosmetics, such as cream, milky lotion, lotion, suspension, gel, powders, packs, sheets, patches, sticks, and cakes. Specific examples of the form of the preparation include, from the viewpoint of treating dermatitis or the like, external medicines such as lotion, shampoos, cream, milky lotion, ointment, and patches, as well as head or body cleansers such as shampoos and body shampoos, and cosmetics for head or body such as rinses, conditioners, treatment, hair packs, hair tonics, hair cream, and lotion, cream, and milky lotion for head or body.

According to an aspect, the preparation of the present disclosure is preferably a skin external preparation such as powders for external use, liniments, lotion, ointment, cream, gel, aerosol sprays, pump sprays, tape, and cataplasm.

The hydrate crystal of the present disclosure can immediately exert excellent antimicrobial activities on fungi. Therefore, according to an aspect, the preparation is used for amelioration of a symptom or a disease caused by a fungus. Here, the term "amelioration" used herein encompasses not only treatment of an established pathology but also prophylaxis in preventing or delaying a pathology to be possibly established in the future. The amelioration in the present disclosure also encompasses improvement of skin disease symptoms or conditions, prevention or delay of exacerbation of skin disease symptoms or conditions, and reversal, prevention or delay of progression of skin disease symptoms or conditions.

Examples of fungus in the present disclosure include fungi of the genus Candida, fungi of the genus Malassezia, fungi of the genus Trichophyton, fungi of the genus Microsporum, fungi of the genus Arthroderma, fungi of the genus Aspergillus, and fungi of the genus Cryptococcus.

The fungi of the genus Candida in the present disclosure are fungi belonging to Saccharomycetaceae, and examples thereof include Candida albicans, and Candida glabrata.

The fungi of the genus Malassezia in the present disclosure are fungi belonging to Malasseziaceae, and examples thereof include Malassezia furfur, Malassezia pachydermatis, Malassezia globosa, Malassezia obtusa, Malassezia restricta, Malassezia sympodialis, Malassezia sloofflae, Malassezia dermatis, Malassezia yamatoensis, Malassezia japonica, and Malassezia nana.

The fungi of the genus Trichophyton in the present disclosure are fungi belonging to Arthrodermataceae, and examples thereof include Trichophyton rubrum, and Trichophyton interdigitale.

The fungi of the genus Microsporum in the present disclosure are fungi belonging to Arthrodermataceae, and examples thereof include Microsporum canis.

The fungi of the genus Arthroderma in the present disclosure are fungi belonging to Arthrodermataceae, and examples thereof include Arthroderma vanbreuseghemii.

The fungi of the genus Aspergillus in the present disclosure are fungi belonging to Trichocomaceae, and examples thereof include Aspergillus fumigatus, and Aspergillus niger.

The fungi of the genus Cryptococcus in the present disclosure are fungi belonging to Tremellaceae, and examples thereof include Cryptococcus neoformans.

According to a preferred aspect, a symptom or a disease caused by a fungus is a skin disease. Examples of skin disease include tinea versicolor, folliculitis, seborrheic dermatitis, psoriasis vulgaris, allergic dermatitis, atopic dermatitis, dermatophytosis, candidiasis, cutaneous malasseziosis, aspergillosis, cryptococcosis and otitis externa, and specific examples thereof include tinea versicolor, folliculitis, seborrheic dermatitis, psoriasis vulgaris, allergic dermatitis, canine Malassezia dermatitis, atopic dermatitis, Malassezia otitis externa, feline Malassezia dermatitis, Malassezia otitis externa, equine Malassezia dermatitis, dermatophytosis, candidiasis, aspergillosis, and cryptococcosis.

The hydrate crystal of the present disclosure can also immediately exert excellent antimicrobial activities on bacteria. Therefore, according to an aspect, the preparation is used for amelioration of a symptom or a disease caused by a bacterium.

Examples of bacterium in the present disclosure include the genus Staphylococcus, the genus Streptococcus, the genus Pasteurella, the genus Escherichia, the genus Pseudomonas, the genus Proteus, and the genus Klebsiella.

The bacteria of the genus Staphylococcus in the present disclosure are bacteria belonging to Staphylococcaceae, and examples thereof include Staphylococcus pseudintermedius, Staphylococcus intermedius, Staphylococcus schleiferi, Staphylococcus delfini, Staphylococcus epidermidis, Staphylococcus xylosus, and Staphylococcus aureus.

The bacteria of the genus Streptococcus in the present disclosure are bacteria belonging to Streptococcaceae, and examples thereof include Streptococcus canis, Streptococcus pyrogenes, Streptococcus suis, and Streptococcus disgalactiae.

The bacteria of the genus Pasteurella in the present disclosure are bacteria belonging to Pasteurellaceae, and examples thereof include Pasteurella multocida.

The bacteria of the genus Escherichia in the present disclosure are bacteria belonging to Enterobacteriaceae, and examples thereof include Escherichia coli.

The bacteria of the genus Pseudomonas in the present disclosure are bacteria belonging to Pseudomonadaceae, and examples thereof include Pseudomonas aeruginosa.

The bacteria of the genus Proteus in the present disclosure are bacteria belonging to Enterobacteriaceae, and examples thereof include Proteus mirabilis.

The bacteria of the genus Klebsiella in the present disclosure are bacteria belonging to Enterobacteriaceae, and examples thereof include Klebsiella pneumoniae.

According to a preferred aspect, a symptom or a disease caused by a bacterium is a skin disease. Examples of the skin disease include pyoderma and otitis externa.

A subject to be administered with the hydrate crystal of the present disclosure is preferably an animal, when the necessity of sterilization of fungi or bacteria, or the like, is taken into consideration. More specific examples of the subj ect to be administered include animals that require suppression of fungi or bacteria growth and amelioration of skin diseases caused by fungi or bacteria. More specific examples thereof include humans, and non-human animals such as dogs, cats and horses. A site to be administered with the hydrate crystal of the present disclosure is not particularly limited, and may be a tissue such as skin, an organ or a cell in which fungi or bacteria of interest are present.

The hydrate crystal of the present disclosure can be effectively applicable for ameliorating a symptom or a disease caused by a fungus or a bacterium, as described above. Therefore, according to another aspect, provided is a method for ameliorating a symptom or a disease caused by a fungus or a bacterium, including administering an effective amount of the hydrate crystal to a subject in need thereof.

Further, according to another more specific aspect of the present disclosure, the hydrate crystal can perform suppressing the growth of or killing a fungus by administering to a subject in need thereof at an effective amount thereof so as to exhibit antifungal activities. Therefore, according to another aspect of the present disclosure, provided is a method for suppressing the growth of or killing a fungus in a subject, including administering an effective amount of the hydrate crystal to the subject in need thereof.

Further, according to another more specific aspect of the present disclosure, the hydrate crystal can perform suppressing the growth of or killing a bacterium by administering to a subject in need thereof at an effective amount so as to exhibit antibacterial activities. Therefore, according to another aspect of the present disclosure, provided is a method for suppressing the growth of or killing a bacterium in a subject, including administering an effective amount of the hydrate crystal to the subject in need thereof.

The method of the present disclosure may be a therapeutic or non-therapeutic method. Specifically, examples of the non-therapeutic method include use for beauty, and non-therapeutic use for health promotion. Therefore, according to a preferred aspect, the method of the present disclosure does not involve medical intervention, that is, therapeutic treatment to individuals.

The effective amount of the hydrate crystal of the present disclosure varies depending on the animal species, sex, age, body weight, condition of a subject to be administered, or other factors, and it is an amount that reduces the growth of a microbe of interest to 50% or less, preferably 40% or less, more preferably 30% or less, further preferably 20% or less, and further more preferably 10% or less of that of the control.

Since the dose, administration route and administration interval of the hydrate crystal of the present disclosure in the above administration varies depending on the animal species, sex, age, body weight, condition of a subject to be administered, or other factors, these cannot uniquely be defined. For example, when the hydrate crystal of the present disclosure is topically administered to an affected area of a human, the daily dosage is generally 0.005 to 350 mg/day, and the preferred weekly dosage is 0.01 to 175 mg/week, per adult human of 60 kg body weight. In addition, when the hydrate crystal of the present disclosure is topically administered to an affected area of a dog, the daily dosage is generally 0.005 to 350 mg/day, and the preferred weekly dosage is 0.01 to 175 mg/week, per adult dog of 10 kg body weight. Further, when the hydrate crystal of the present disclosure is topically administered to an affected area of a cat, the daily dosage is generally 0.002 to 140 mg/day, and the preferred weekly dosage is 0.004 to 70 mg/week, per adult cat of 4 kg body weight.

Also, according to another aspect of the present disclosure, provided is use of the hydrate crystal, in production of a pharmaceutical, a quasi-drug, a veterinary drug or a cosmetic. In addition, according to another aspect of the present disclosure, provided is use of the hydrate crystal, in production of an antimicrobial agent.

Further, according to another aspect of the present disclosure, provided is use of the hydrate crystal, in production of an antifungal agent. Furthermore, according to another preferred aspect of the present disclosure, provided is use of the hydrate crystal, in production of a preparation for amelioration of a symptom or a disease caused by a fungus. In addition, according to another preferred aspect of the present disclosure, the symptom or disease is a skin disease. Also, according to another preferred aspect of the present disclosure, the symptom or disease is tinea versicolor, folliculitis, seborrheic dermatitis, psoriasis vulgaris, allergic dermatitis, atopic dermatitis, dermatophytosis, candidiasis, cutaneous malasseziosis, aspergillosis, cryptococcosis, or otitis externa. Moreover, according to another preferred aspect of the present disclosure, the above use is for beauty or non-therapeutic use.

Further, according to another aspect of the present disclosure, provided is use of the hydrate crystal, in production of an antibacterial agent. Furthermore, according to another preferred aspect of the present disclosure, provided is use of the hydrate crystal, in production of a preparation for amelioration of a symptom or a disease caused by a bacterium. In addition, according to another preferred aspect of the present disclosure, the symptom or disease is a skin disease. Also, according to another preferred aspect of the present disclosure, the symptom or disease is pyoderma or otitis externa. Moreover, according to another preferred aspect of the present disclosure, the above use is for beauty or non-therapeutic use.

Further, according to another aspect of the present disclosure, provided is the hydrate crystal for use as antifungal agent. Furthermore, according to another preferred aspect of the present disclosure, provided is the hydrate crystal for amelioration of a symptom or a disease caused by a fungus. In addition, according to another preferred aspect of the present disclosure, the symptom or disease is a skin disease. Also, according to another preferred aspect of the present disclosure, the symptom or disease is tinea versicolor, folliculitis, seborrheic dermatitis, psoriasis vulgaris, allergic dermatitis, allergic dermatitis, atopic dermatitis, dermatophytosis, candidiasis, cutaneous malasseziosis, aspergillosis, cryptococcosis, or otitis externa. Moreover, according to another preferred aspect of the present disclosure, the antifungal agent is a pharmaceutical or a veterinary drug.

Further, according to another aspect of the present disclosure, provided is the hydrate crystal for use as antibacterial agent. Furthermore, according to another preferred aspect of the present disclosure, provided is the hydrate crystal for amelioration of a symptom or a disease caused by a bacterium. In addition, according to another preferred aspect of the present disclosure, the symptom or disease is a skin disease. Also, according to another preferred aspect of the present disclosure, the symptom or disease is pyoderma or otitis externa. Moreover, according to another preferred aspect of the present disclosure, the antibacterial agent is a pharmaceutical or a veterinary drug.

Further, according to an aspect, the followings are provided.
[1] Ahydrate crystal of 5-chloro-4-(3-chloro-4-methylphenyl)-1H-imidazole-2-carbonitrile.
[2] The hydrate crystal according to [1], in which the hydrate crystal is a monohydrate.
[3] The hydrate crystal according to [1] or [2], in which a powder X-ray diffraction pattern measured using Cu-Kα as a radiation source at a tube voltage of 40 kV and a tube current of 40 mA has at least top 5 peaks with highest intensity at diffraction angles (2θ ± 0.2°) of 9.8, 12.8, 14.6, 16.0, 19.5, 24.2, 25.3, 25.7, 26.7, 27.7, 28.5, 28.8, 29.5, 30.3, 32.3, 33.1, 34.1, 35.7, 36.4, 37.4, 39.5, 40.1, 43.3, or
   44.3.
[4] The hydrate crystal according to [3], in which a powder X-ray diffraction pattern measured using Cu-Kα as a radiation source at a tube voltage of 40 kV and a tube current of 40 mA has at least top 7 peaks with highest intensity at diffraction angles (2θ ± 0.2°) of 9.8, 12.8, 14.6, 16.0, 19.5, 24.2, 25.3, 25.7, 26.7, 27.7, 28.5, 28.8, 29.5, 30.3, 32.3, 33.1, 34.1, 35.7, 36.4, 37.4, 39.5, 40.1,
   43.3, or 44.3.
[5] The hydrate crystal according to [4], in which a powder X-ray diffraction pattern measured using Cu-Kα as a radiation source at a tube voltage of 40 kV and a tube current of 40 mA has at least top 9 peaks with highest intensity at diffraction angles (2θ ± 0.2°) of 9.8, 12.8, 14.6, 16.0, 19.5, 24.2, 25.3, 25.7, 26.7, 27.7, 28.5, 28.8, 29.5, 30.3, 32.3, 33.1, 34.1, 35.7, 36.4, 37.4, 39.5, 40.1,
   43.3, or 44.3.
[6] The hydrate crystal according to [1] or [2], in which a powder X-ray diffraction pattern measured using Cu-Kα as a radiation source at a tube voltage of 40 kV and a tube current of 40 mA has at least top 5 peaks with highest intensity at diffraction angles (2θ ± 0.2°) of 9.8, 12.8, 14.6, 16.0, 19.5, 25.3, 25.7, 26.7, or 29.5.
[7] The hydrate crystal according to [6], in which a powder X-ray diffraction pattern measured using Cu-Kα as a radiation source at a tube voltage of 40 kV and a tube current of 40 mA has at least top 7 peaks with highest intensity at diffraction angles (2θ ± 0.2°) of 9.8, 12.8, 14.6, 16.0, 19.5, 25.3, 25.7, 26.7, or 29.5.
[8] The hydrate crystal according to any of [1] to [7], in which a moisture content measured by a Karl Fischer method (volumetric titration method) is 6.5 to 6.8% by mass.
[9] An antimicrobial agent, including the hydrate crystal according to any of [1] to [8].
[10] The antimicrobial agent according to [9], in which a microbe is a fungus.
[11] The antimicrobial agent according to [10], in which the fungus is at least one selected from the genus Candida, the genus Malassezia, the genus Trichophyton, the genus Microsporum, the genus Arthroderma, the genus Aspergillus, and the genus Cryptococcus.
[12] The antimicrobial agent according to [11], in which the fungus is at least one selected from the genus Candida, the genus Trichophyton, the genus Microsporum, the genus Arthroderma, the genus Aspergillus, and the genus Cryptococcus.
[13] The antimicrobial agent according to [11], in which the fungus is at least one selected from the genus Malassezia, the genus Microsporum, and the genus Arthroderma.
[14] The antimicrobial agent according to any of [10] to [13], for ameliorating a symptom or a disease caused by the above fungi.
[15] The antimicrobial agent according to [14], in which the symptom or the disease is a skin disease.
[16] The antimicrobial agent according to [14] or [15], in which the symptom or disease is tinea versicolor, folliculitis, seborrheic dermatitis, psoriasis vulgaris, allergic dermatitis, atopic dermatitis, dermatophytosis, candidiasis, cutaneous malasseziosis, aspergillosis, cryptococcosis, or otitis externa.
[17] The antimicrobial agent according to [14] to [16], in which the symptom or disease is cutaneous malasseziosis, dermatophytosis, candidiasis, aspergillosis, or cryptococcosis.
[18] The antimicrobial agent according to [9], in which the microbe is a bacterium.
[19] The antimicrobial agent according to [18], in which the bacterium is at least one selected from the genus Staphylococcus, the genus Streptococcus, the genus Pasteurella, the genus Escherichia, the genus Pseudomonas, the genus Proteus, and the genus Klebsiella.
[20] The antimicrobial agent according to [18] or [19], for ameliorating a symptom or a disease caused by the bacteria.
[21] The antimicrobial agent according to [20], in which the symptom or the disease is a skin disease.
[22] The antimicrobial agent according to [20] or [21], in which the symptom or the disease is pyoderma or otitis externa.

Further, according to another aspect of the present disclosure, the followings are provided.
[23] Ahydrate crystal of 5-chloro-4-(3-chloro-4-methylphenyl)-1H-imidazole-2-carbonitrile.
[24] The hydrate crystal according to claim 1, in which the hydrate crystal is a monohydrate crystal.
[25] The hydrate crystal according to [23], in which a powder X-ray diffraction pattern measured using a Cu-Kα radiation source has at least one peak at a diffraction angle (2θ ± 0.2°) of 9.8, 12.8, 14.6, 16.0, 19.5, 24.2, 25.3, 25.7, 26.7, 27.7, 28.5, 28.8, 29.5, 30.3, 32.3, 33.1, 34.1, 35.7, 36.4, 37.4, 39.5, 40.1, 43.3, or 44.3.
[26] The hydrate crystal according to [23], in which a powder X-ray diffraction pattern measured using the Cu-Kα radiation source has at least one peak at a diffraction angle (2θ ± 0.2°) of 9.8, 12.8, 14.6, 16.0, 19.5, 25.3, 25.7, 26.7, or 29.5.
[27] The hydrate crystal according to [25] or [26], in which the at least one peak is at least top 5 peaks with highest intensity.
[28] The hydrate crystal according to [23], in which at least one endothermic peak falls within a range selected from 93 to 100°C and 135 to 145°C in thermogravimetric-differential thermal analysis (TG-DTA) or differential scanning calorimetry analysis (DSC).
[29] The hydrate crystal according to [23], in which an infrared absorption spectrum measured by a potassium bromide disk method has at least one peak at a position (cm-1) selected from 835 ± 2, 860 ± 2, 1014 ± 2, 1053 ± 2, 1157 ± 2, 1315 ± 2, 1427 ± 2, 1454 ± 2, 1500 ± 2, 1581 ± 2, 1654 ± 2 and 2245 ± 2.
[30] The hydrate crystal according to [23], in which a Raman spectroscopy spectrum has at least one peak at a position (cm-1) selected from 1021 ± 2, 1152 ± 2, 1240 ± 2, 1281 ± 2, 1379 ± 2, 1409 ± 2, 1424 ± 2, 1451 ± 2, 1507 ± 2, 1573 ± 2, 1607 ± 2 and 2241 ± 2.
[31] The hydrate crystal according to [23], in which a moisture content measured by a Karl Fischer method (volumetric titration method) is 6.5 to 6.8% by mass.
[32] A preparation comprising the hydrate crystal according to [23] and a pharmacologically acceptable additional ingredient.
[33] The preparation according to [32], in which the preparation is a pharmaceutical, a quasi-drug, a veterinary drug, or a cosmetic.
[34] The preparation according to [32], in which the preparation is an antimicrobial agent.
[35] The preparation according to [32], in which the preparation is an antifungal agent or an antibacterial agent.
[36] The preparation according to [32], in which the preparation is for ameliorating a symptom or a disease caused by a fungus or bacterium.
[37] The preparation according to [36], in which the symptom or the disease is a skin disease.
[38] The preparation according to [34] or [35], for use against at least one fungus selected from the genus Candida, the genus Malassezia, the genus Trichophyton, the genus Microsporum, the genus Arthroderma, the genus Aspergillus and the genus Cryptococcus.
[39] The preparation according to [34] or [35], for use against at least one bacterium selected from the genus Staphylococcus, the genus Streptococcus, the genus Pasteurella, the genus Escherichia, the genus Pseudomonas, the genus Proteus, and the genus Klebsiella.
[40] Use of a hydrate crystal of 5-chloro-4-(3-chloro-4-methylphenyl)-1H-imidazole-2-carbonitrile, in production of a pharmaceutical, a quasi-drug, a veterinary drug or a cosmetic.
[41] Use of a hydrate crystal of 5-chloro-4-(3-chloro-4-methylphenyl)-1H-imidazole-2-carbonitrile, in production of an antimicrobial agent.
[42] A method for ameliorating a symptom or a disease caused by a fungus or a bacterium, including:
   administering to a subject in need thereof, an effective amount of a hydrate crystal of 5-chloro-4-(3-chloro-4-methylphenyl)-1H-imidazole-2-carbonitrile.

### Examples

Test Examples relating to the present disclosure will now be described; however, the present disclosure is not limited thereto.

### Production Example 1: Synthesis of Hydrate Crystals of 5-Chloro-4-(3-Chloro-4-Methylphenyl)-1H-Imidazole-2-Carbonitrile

Anhydrous crystals (CAS No. 120118-82-3, MS: 252.1 [M+H]+) were produced according to the same technique as described in Japanese Patent Laid-Open No. 8-225539, except for that raw material compounds corresponding to a production target were used.

250 g of the resulting anhydrous crystals were placed in a paper bag and left to stand still in a stability chamber set to a temperature of 60°C and a relative humidity of 80% until the crystals reached a saturated state with no more change in weight. The change in weight stopped after 20 days of standing still, and then the crystals were collected to yield 267 g of hydrate crystals.

### Production Example 2: Synthesis of Hydrate Crystals of 5-Chloro-4-(3-Chloro-4-Methylphenyl)-1H-Imidazole-2-Carbonitrile

Anhydrous crystals were produced in the same manner in Production Example 1. 100 g of the resulting anhydrous crystals were placed in a 500 mL four-necked flask to dissolve in 238 g of methanol. 300 g of H2O were added dropwise over an hour, and then the mixture was stirred for 15 hours. Filtration under reduced pressure was performed at 20 to 25°C, and the crystals were collected by washing with a cleansing solution (methanol : H2O = 40 g : 50 g) for a reaction container. The collected crystals were air dried at 25°C and a relative humidity of 40% for 15 hours to yield 98 g of hydrate crystals.

### Test Example 1

### 1-1: Powder X-Ray Diffraction (XRD)

The powder X-ray diffraction patterns of the hydrate crystals obtained in Production Examples 1 and 2, and the anhydrous crystals used as a raw material in Production Example 1 were measured under the following conditions. For the measurement, a high-performance X-ray diffraction apparatus (D8 DISCOVER, manufactured by Bruker AXS GmbH) was used.
X-ray source: Cu-Kα
Tube voltage: 40 kV
Tube current: 40 mA
Measurement temperature: Room temperature
20: 5° to 60°
Step width: 0.02°
Scanning speed: 0.2 s/point

Figures 1 and 2 show the powder X-ray diffraction patterns of the hydrate crystals obtained in Production Examples 1 and 2, respectively.

In the powder X-ray diffraction pattern of Figure 1, major peaks were observed at positions of 2θ of 9.8,
12.8, 14.6, 16.0, 19.5, 24.2, 25.3, 25.7, 26.7, 27.7, 28.5, 28.8, 29.5, 30.3, 32.3, 33.1, 34.1, 35.7, 36.4, 37.4,39.5,40.1,43.3,44.3.

The 5 peaks of high intensity had 2θ of 25.7, 9.8, 12.8, 26.7, and 25.3 in order of strength. In addition, the 7 peaks of high intensity had 2θ of 25.7, 9.8, 12.8, 26.7, 25.3, 19.5, and 16.0 in order of strength. Further, the 9 peaks of high intensity had 2θ of 25.7, 9.8, 12.8, 26.7, 25.3, 19.5, 16.0, 14.6, and 27.7 in order of strength.

In the powder X-ray diffraction pattern of Figure 2, major peaks were observed at positions of 2θ of 9.8, 12.8, 14.6, 16.0, 19.5, 24.2, 25.3, 25.7, 26.7, 27.7, 28.5, 28.8, 29.5, 30.3, 32.3, 33.1, 34.1, 35.7, 36.4, 37.4, 39.5, 40.1, 43.3, 44.3, as in Production Example 1. However, the 5 peaks of high intensity had 2θ of 9.8, 12.8, 16.0, 19.5, and 25.7 in order of strength. In addition, the 7 peaks of high intensity had 2θ of 9.8, 12.8, 16.0, 19.5, 25.7, 26.7, and 14.6 in order of strength. Further, the 9 peaks of high intensity had 2θ of 9.8, 12.8, 16.0, 19.5, 25.7, 26.7, 14.6, 29.5, and 25.3 in order of strength.

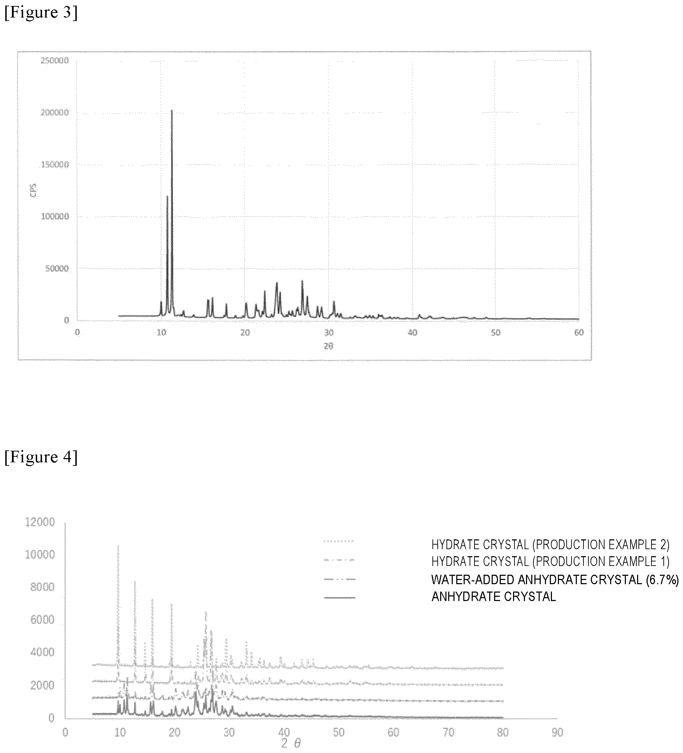

Figure 3 shows the powder X-ray diffraction patterns of the anhydrous crystals used as a raw material in Production Example 1. In the powder X-ray diffraction pattern of Figure 3, major peaks were observed at positions of 2θ of 10.0, 10.7, 11.3, 15.6, 16.1, 20.1, 21.2, 22.3, 23.8, 26.8, 27.3, 28.6, 29.1, and 30.6, confirming that the crystal structure is clearly different from those of the hydrate crystals in Figures 1 and 2. The 5 peaks of high intensity had 2θ of 11.3, 10.7, 26.8, 23.8, and 22.3 in order of strength. In addition, the 7 peaks of high intensity had 2θ of 11.3, 10.7, 26.8, 23.8, 22.3, 27.3, and 16.1 in order of strength. Further, the 9 peaks of high intensity had 2θ of 11.3, 10.7, 26.8, 23.8, 22.3, 27.3, 16.1, 15.6, and 30.6 in order of strength.

### 1-2: Comparison of Powder X-Ray Diffraction Patterns of Water-added Anhydrate Crystal, Anhydrate Crystal and Hydrate Crystal

The XRD chart of the water-added anhydrate crystals (anhydrate crystals in the presence of water of adhesion) was obtained, and the powder X-ray diffraction pattern of the water-added sample was compared with the powder X-ray diffraction patterns of the anhydrate crystals and hydrate crystals.
Specifically, the anhydrate crystal sample was added with water immediately before measurement, such that the weight ratio was 6.7% (moisture content corresponding to a monohydrate), and mixed to be homogeneous, and then the measurement was performed by a powder X-ray diffraction method in a similar manner to that in Test Example 1-1.

Figure 4 is a comparison graph of XRD charts for a water-added anhydrate crystal, an anhydrate crystal, and hydrate crystals (Production Example 1 and Production Example 2).

The powder X-ray diffraction angle 2θ + 0.2 of the water-added anhydrate crystals exhibited a pattern consisting of 10.0, 10.7, 11.3, 15.6, 16.1, 20.1, 21.2, 22.3, 23.8, 26.8, 27.3, 28.6, 29.1, and 30.6, which was consistent with the pattern of the anhydrate crystals. In other words, the anhydrate crystals to which a moisture content corresponding to an monohydrate was added had a similar pattern to that of dried anhydrate crystals. This revealed that the water in the water-added anhydrate crystals was water of adhesion, and the moisture contained in the hydrate crystals was not water of adhesion, but water of crystallization.

### 1-3: Thermogravimetric-Differential Thermal Analysis

By subjecting powder test substances to thermal analysis such as thermogravimetric-differential thermal analysis (TG-DTA) or differential scanning calorimetry analysis (DSC), anhydrate and hydrate are distinguishable by using the presence or absence of endothermic peaks derived from hydrated water as an index. In addition, the thermogravimetric-differential thermal analysis can simultaneously measure the change in weight, and therefore, the content of water of crystallization can be examined. In order to evaluate crystals of 5-chloro-4-(3-chloro-4-methylphenyl)-1H-imidazole-2-carbonitrile by making use of such characteristics of the thermal analysis, the following measurement was performed.

The thermogravimetric-differential thermal analysis was performed by placing about 10 mg of test substances in an aluminum pan for measurement and setting to an apparatus. Measurement conditions such as the temperature increase rate were as follows, and data was taken in from the measurement initial temperature to 300°C.

Measurement container: Aluminum pan
Measurement atmosphere: Air
Aeration rate: 100 mL/min
Temperature increase rate: 20°C/min
Sample observation simultaneous thermal analyzer combining thermogravimetry - differential thermal analysis: Thermo plus EVO2 TG-DTA8122/C (manufactured by Rigaku Corporation)

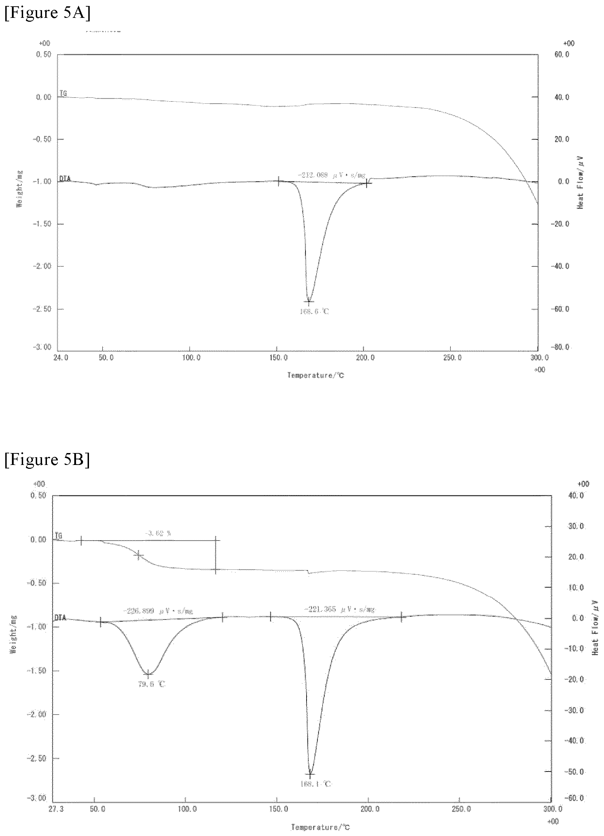

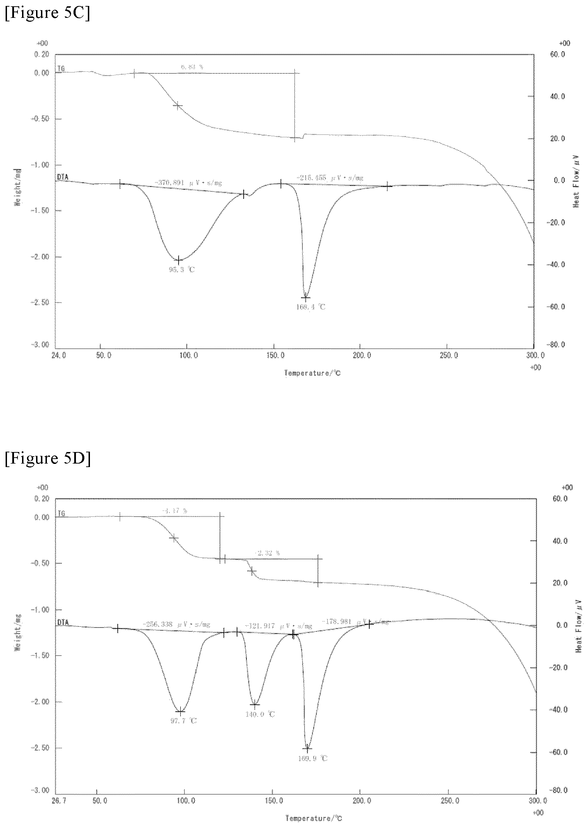

Figure 5Ais a TG-DTA chart of the anhydrate crystal.

Figure 5B is a TG-DTA chart of the water-added anhydrate crystal.

Figure 5C is a TG-DTA chart of the hydrate crystal (Production Example 1).

Figure 5D is a TG-DTA chart of the hydrate crystal (Production Example 2).

In the TG-DTA charts, all of the anhydrate crystals and hydrate crystals had endothermic peaks, which indicate decomposition of the compounds themselves, at the vicinity of 168°C. In addition, the hydrate crystals had endothermic peaks associated with desorption of water of crystallization: an endothermic peak at the vicinity of 95°C in Production Example 1 and two endothermic peaks at the vicinity of 98°C and 140°C in Production Example 2. These endothermic peaks associated with desorption of hydrated water were linked with weight loss in TG-DTA, which involved weight loss of about 6.7% in total. Since the weight loss of about 6.7% in Production Example 1 and Production Example 2 was consistent with the theoretical value of the moisture content of a monohydrate, Production Example 1 and Production Example 2 were confirmed to be monohydrate crystals.

Although Production Example 1 and Production Example 2 were both monohydrate crystals, the patterns of the TG-DTA charts thereof differed. In Production Example 1, crystals were obtained by causing the anhydrate crystals to absorb moisture, whereas in Production Example 2, crystals were obtained by crystallizing as hydrates; therefore, it is considered that differences in the growth degree and orientation of the crystal faces, and the like occurred, causing the patterns of the TG-DTA charts to be different.

Note that the endothermic peak of the water-added anhydrate crystal was at the vicinity of 80°C, which was lower than those of Production Example 1 and Production Example 2. It is commonly known that the desorption of water of adhesion occurs at a temperature lower than that for desorption of water of crystallization, and this finding is consistent with the fact that the endothermic peak of the water-added anhydrate crystal (anhydrate crystal in the presence of water of adhesion) was lower than those of Production Example 1 and Production Example 2 (monohydrate crystals containing water of crystallization).

### 1-4: Observation of Anhydrate Crystal and Hydrate Crystal by Scanning Electron Microscope

The anhydrate crystals and hydrate crystals (Production Example 1 and Production Example 2) of 5-chloro-4-(3-chloro-4-methylphenyl)-1H-imidazole-2-carbonitrile within a month after obtaining the crystals were observed by a scanning electron microscope (S-3200N, manufactured by Hitachi High-Technologies Corporation) to compare the crystal structures thereof.

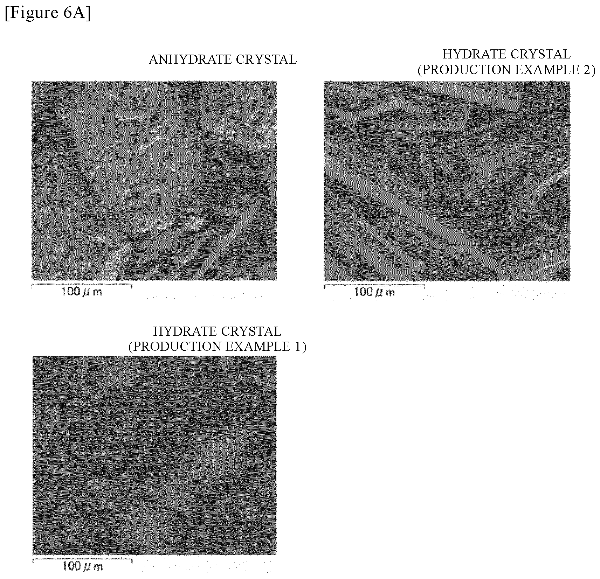

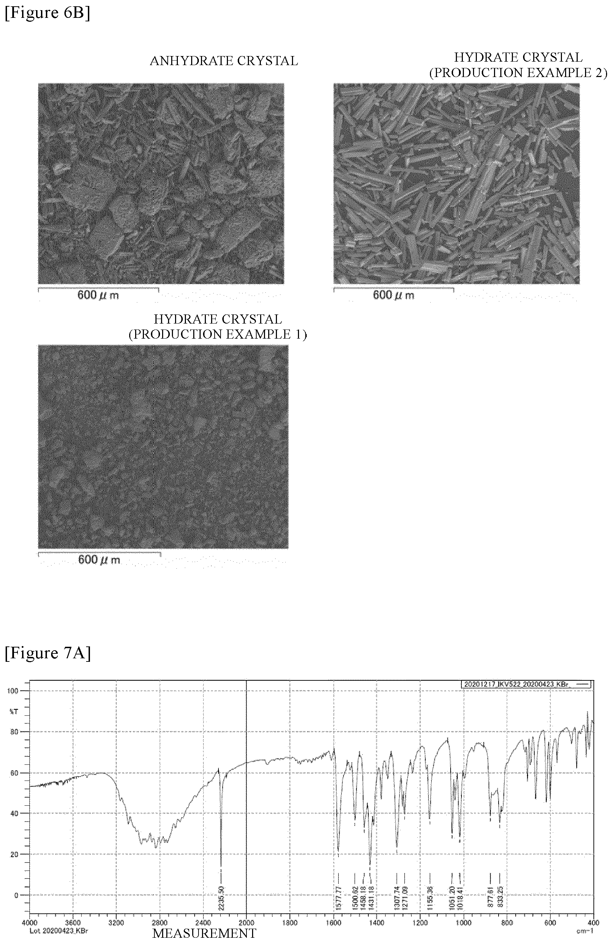

The results were as shown in Figures 6A and 6B.

Figure 6Ais photographs of the anhydrate crystals, the hydrate crystals (Production Examples 1) and the hydrate crystals (Production Examples 2) taken at 500x magnification with the scanning electron microscope.

Figure 6B is photographs of the anhydrate crystals, the hydrate crystals (Production Examples 1) and the hydrate crystals (Production Examples 2) taken at 100x magnification with the scanning electron microscope.

The anhydrate crystals were not homogeneous, and something like aggregates containing columnar crystals were observed. On the other hand, in the hydrate crystals (Production Examples 2), every columnar crystal grew significantly, and aggregates as shown in the anhydrate crystals were not observed. In the hydrate crystals (Production Examples 1), massive (including substantially rectangular parallelepiped shape) crystal grains of uneven size were observed. In the hydrate crystals (Production Examples 1), it considered that homogeneous crystal structure was not able to be observed due to the fact that the structure of the original anhydrate was collapsed in the hydrating process.

### 1-5: Comparison of Anhydrate Crystal and Hydrate Crystal by Infrared Absorption Spectra

The anhydrate crystals and hydrate crystals (Production Example 2) of 5-chloro-4-(3-chloro-4-methylphenyl)-1H-imidazole-2-carbonitrile were measured with an infrared spectrophotometer in the following manner, and the infrared absorption spectra thereof were compared.

About 1 mg of the test substance was ground in an agate mortar, 0.20 g of potassium bromide were added thereto to obtain a mixture, about 10 mg of which were weighed out and pressure tabletted with a diameter of 3 mm by a tableting machine. The infrared absorption spectrum of the produced sample was measured under the following conditions in a wave range of 4000 to 400 cm-1.

Measurement method: Potassium Bromide Disk Method
The number of accumulations: 32 times
Resolution: 4 cm-1
Fourier transform infrared spectrophotometer IRAffinity-1S (SHIMADZU CORPORATION)

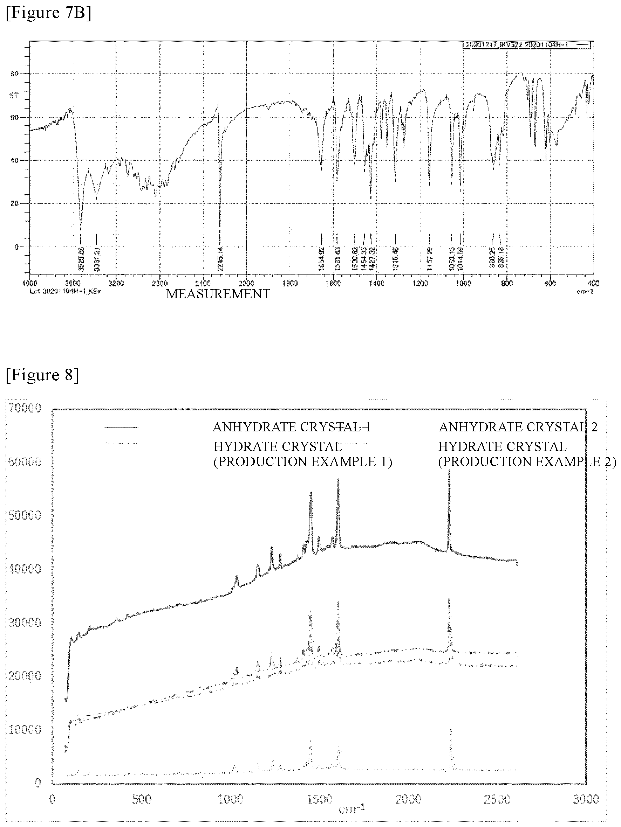

The results were as shown in Figure 7A and Figure 7B, and Table 1.

Figure 7Ais an infrared absorption spectrum of the anhydrate crystal.

Figure 7B is an infrared absorption spectrum of the hydrate crystal (Production Examples 2).

Table 1 shows the top 12 peaks (in a range of 2400 to 600 cm-1) in descending order of peak intensity in the infrared absorption spectra of the anhydrate crystals and the hydrate crystals (Production Example 2)).

**[Table 1]**

| Anhydrate crystal (cm-1) | Hydrate crystal (cm-1) |
|---|---|
| 2235 | 2245 |
| 1577 | 1654 |
| 1500 | 1581 |
| 1458 | 1500 |
| 1431 | 1454 |
| 1307 | 1427 |
| 1271 | 1315 |
| 1155 | 1157 |
| 1051 | 1053 |
| 1018 | 1014 |
| 877 | 860 |
| 833 | 835 |

The above results suggested that anhydrate crystals and hydrate crystals can also be distinguishable with infrared absorption spectra, and crystal forms changed by hydrating.

### 1-6: Comparison of Anhydrate Crystal and Hydrate Crystal by Raman Spectroscopy Spectra

The anhydrate crystals and hydrate crystals of 5-chloro-4-(3-chloro-4-methylphenyl)-1H-imidazole-2-carbonitrile were measured with a Raman spectroscopy system in the following manner, and the Raman spectroscopy spectra thereof were compared.

To a glass sample holder, test substances (anhydrate crystal 1, anhydrate crystal 2, hydrate crystal (Production Example 1), hydrate crystal (Production Example 2) were thinly applied, the thus obtained was set to the Raman spectroscopy system and irradiated with a laser after an arbitrary point was specified on a microscope to obtain Raman spectroscopy spectra. Note that the anhydrate crystal 1 and the anhydrate crystal 2 are anhydrate crystals produced in a different time point.

Laser wave length: 532 nm
Diffraction grating 600 gr/mm
Spatial resolution: 350 nm
Wave number resolution: 0.5 cm-1
Raman spectroscopy system: RAMANtouch VIS-ICS-S (Nanophoton Corporation)

The results were as shown in Figure 8 and Table 2.

Figure 8 is a comparison graph of Raman spectroscopy spectra of the anhydrate crystal 1, the anhydrate crystal 2, the hydrate crystal (Production Examples 1) and the hydrate crystal (Production Examples 2).

Table 2 shows the top 12 peaks (in a range of 2400 to 600 cm-1) in descending order of peak intensity in the Raman spectroscopy spectra of the anhydrate crystal 1, the anhydrate crystal 2, the hydrate crystal (Production Examples 1) and the hydrate crystal (Production Examples 2).

**[Table 2]**

| Anhydrate crystal 1 (cm-1) | Anhydrate crystal 2 (cm-1) | Hydrate crystal (Production Example 1) (cm-1) | Hydrate crystal (Production Example 2) (cm-1) |
|---|---|---|---|
| 2230 | 2230 | 2241 | 2241 |
| 1605 | 1605 | 1607 | 1607 |
| 1571 | 1571 | 1573 | 1573 |
| 1500 | 1500 | 1507 | 1507 |
| 1453 | 1453 | 1451 | 1451 |
| 1438 | 1438 | 1424 | 1424 |
| 1409 | 1409 | 1409 | 1409 |
| 1376 | 1376 | 1379 | 1379 |
| 1276 | 1276 | 1281 | 1281 |
| 1231 | 1231 | 1240 | 1240 |
| 1149 | 1149 | 1152 | 1152 |
| 1036 | 1036 | 1021 | 1021 |

Raman shifts were observed between the anhydrate crystals and the hydrate crystals even for the peaks derived from the same functional group. The above results suggested that anhydrate crystals and hydrate crystals can also be distinguishable with Raman spectroscopy spectra, and crystal forms changed by hydrating.

### Test Example 2: Karl Fischer Moisture Content Measurement (Volumetric Titration Method)

The moisture content rates of the hydrate crystals obtained in Production Examples 1 and 2, and the anhydrous crystals used as a raw material in Production Example 1 were measured with a Karl Fischer moisture meter (CA-310, manufactured by Nittoseiko Analytech Co., Ltd.). The test was conducted in conformance with the description in The Japanese Pharmacopoeia, 18th Edition, "2.48 Water Determination (Karl Fischer Method)", Volumetric titration. The measurement was performed in an atmosphere of 25°C, with the conditions set to a forced delay time of 2 minutes after sample administration, a minimum amount to be titrated of 2 µL, and an end point voltage of 140 mV More specifically, it was as follows.

First, into a measuring flask, methanol for moisture measurement (Aquamicron Dehydrated Solvent GEX, manufactured by Mitsubishi Chemical Corporation) was loaded, and 30 mg of water was added thereto to titrate the mixture with a Karl Fischer reagent (Aquamicron SS-Z, manufactured by Mitsubishi Chemical Corporation). After titration, a titer (mg/mL) of the reagent was determined from the amount titrated. Next, about 0.1 g of a measurement sample was precisely weighed and added into the measuring flask to titrate the mixture with a Karl Fischer reagent. The moisture content (w/w%) in the sample was calculated from the amount titrated and the previously determined titer.

The respective Karl Fischer moisture content of the hydrate crystal of Production Example 1 the hydrate crystal of Production Example 2, the anhydrous crystal were 6.6 wt%, 6.7 wt%, and 0.1 wt%. Since the molecular weight of 5-chloro-4-(3-chloro-4-methylphenyl)-1H-imidazole-2-carbonitrile is 252.10, and the molecular weight of water is 18.00, it is considered that the hydrate crystals of Production Examples 1 and 2 are monohydrate.

### Test Example 3: Moisture absorption Test

An anhydrous crystal sample was placed in a container (glass weighing bottle or paper bag) in such a manner that it was in direct contact with the damp. The samples were preserved under the respective conditions of temperature and humidity (25°C/80% RH, and 40°C/80% RH) as shown in Table 1, and the change in moisture content was observed through the change in weight or Karl Fischer moisture measurement. In addition, a sample for the hydrate crystal (Production Example 1) was also preserved at 60°C/80% RH, and the change in moisture content was observed through the change in weight or Karl Fischer moisture measurement. The results are all shown in Table 3.

**[Table 3]**

| Preserving conditions (temperature/relative humidity) | Preserving container | Storage period | Weight change | Moisture content |
|---|---|---|---|---|
| 25°C/80% RH (Anhydrate crystal ) | Paper bag | 0 days | - | 0.1% |
| | | 14 days | 0.6% | - |
| | | 28 days | 2.2% | - |
| | | 35 days | 3.3% | - |
| | | 43 days | 4.2% | - |
| | | 49 days | 4.7% | - |
| 40°C/75% RH (Anhydrate crystal ) | Weighing bottle | 0 days | - | 0.1% |
| | | 7 days | 3.5% | - |
| | | 14 days | 5.0% | 5.7% |
| | | 21 days | 6.4% | - |
| | | 28 days | 6.7% | 6.1% |
| 60°C/80% RH (Hydrate crystal (Production Example 1)) | Paper bag | 0 days | - | 0.1% |
| | | 4 days | 6.4% | - |
| | | 7 days | 6.4% | - |
| | | 14 days | 6.5% | - |
| | | 20 days | 6.5% | 6.7% |
| | | 29 days | 6.5% | - |
| | | 40 days | 6.5% | - |
| | | 61 days | 6.5% | 6.6% |

A weight increase that is considered to be moisture absorption was observed under the conditions of 25°C/80% RH from Table 1. Under their accelerated conditions of 40°C/75% RH and 60°C/80% RH, weight increases that are considered to be moisture absorption were observed more rapidly. From these, the anhydrous crystal of 5-chloro-4-(3-chloro-4-methylphenyl)-1H-imidazole-2-carbonitrile has relatively high hygroscopicity.

The sample after storage at 60°C/80% RH for 20 days corresponds to the hydrate of Production Example 1 described above. The sample did not exhibit any more change in weight even after 61 days of storage (41 days past after generation of the hydrate), and the moisture content was kept between 6.6 to 6.7% with no increase. From these, it was confirmed that 5-chloro-4-(3-chloro-4-methylphenyl)-1H-imidazole-2-carbonitrile does not absorb moisture as much as the monohydrate, and only if it is made into a monohydrate, it can be stably present even in a high-temperature and high-humidity environment. On the other hand, as described above, the anhydrous crystal had a weight increase of 4.7% under the conditions of 25°C/80% RH after 49 days of storage, and a weight increase of 6.7% and Karl Fischer moisture content of 6.1% at 40°C/75% RH after 28 days of storage. These indicate that more weight increases are expected with continued storage, which cannot to be said to be stable.

### Test Example 4: Drying Test

The hydrate crystal of Production Example 1 was stored at 25°C under a dried condition (in the presence of silica gel) for a period shown in Table 2. The Karl Fischer moisture content after storage was measured, and the change in moisture content was observed. The results are all shown in Table 4.

**[Table 4]**

| Preserving conditions | Preserving container | Storage period | Karl Fischer moisture content |
|---|---|---|---|
| 25°C, dried | Weighing bottle | 0 days | 6.5% |
| | | 14 days | 6.7% |
| | | 29 days | 6.7% |
| | | 38 days | 6.7% |
| | | 53 days | 6.7% |

Table 4 confirmed that the hydrate crystal of Production Example 1 did not have any change in moisture content, and it can be stably present even after storage for a long period of time in a dried environment.

Since the anhydrous crystal of 5-chloro-4-(3-chloro-4-methylphenyl)-1H-imidazole-2-carbonitrile has hygroscopicity, a storage container, a storage location, an expiry date, use of dividing bag for standard product for testing and the like are restricted. The results of Test Examples 3 and 4 revealed that making 5-chloro-4-(3-chloro-4-methylphenyl)-1H-imidazole-2-carbonitrile into a hydrate crystal allows it to be stably present in either case of a high-humidity environment or a dried environment, and thereby the handling of it as an active pharmaceutical ingredient becomes easy.

### Test Example 5: Test of Triboelectrification Properties

The triboelectrification properties on the hydrate crystals obtained in Production Example 1 and Production Example 2, and the anhydrous crystals used as a raw material in Production Example 1 were evaluated.

First, the sample was loaded into a levelled stainless steel petri dish having ϕ50 mm × a height of 15 mm, and then it was statically eliminated with a static eliminator (STABLO AP, manufactured by SHIMADZU CORPORATION). The surface potential of the sample was measured with a surface electrometer (SV-77A, manufactured by NIPPON STATIC CO., LTD.) that had previously been zero-point adjusted, to confirm that it was statically eliminated (within -1 to +1 kV).

Next, the sample surface of the petri dish loaded with the sample was scraped back and forth 20 times with a stainless steel metal plate for 10 seconds to charge the sample surface. After that, the petri dish loaded with the charged sample was placed on a metal plate (30 cm × 30 cm) connected with a ground wire.

The surface potential of the sample was measured over time for 20 minutes with the above surface electrometer. The triboelectrification properties were evaluated from the initial surface potential. In addition, the retention rate was calculated from the surface potential after 20 minutes and the initial surface potential to evaluate charge attenuation properties. Note that the test was conducted in an electrostatic measurement chamber (Tabai ESPEC CORP) set to 25°C and 50% RH, and the surface potential measurement was conducted at a distance of 1 cm from the sample surface. The results are shown in Table 5.

**[Table 5]**

| | Time | Anhydrate crystal | Hydrate crystal (Production Example 1) | Hydrate crystal (Production Example 2) |
|---|---|---|---|---|
| Electrification voltage (kv) | 0 (Minutes) | -5.42 | -3.00 | -2.30 |
| | 20 (Minutes) | -5.39 | -2.79 | -1.98 |
| Retention rate (%) | | 99.4 | 93.0 | 86.1 |

Table 5 revealed that making anhydrous crystals into hydrate crystals causes electrification voltage to be significantly decreased. In addition, the potential retention rate of the anhydrous crystal has almost no change at 20 minutes later, whereas that of the hydrate crystal of Production Example 1 decreased by 7% and that of the hydrate crystal of Production Example 2 decreased by 14%. Accordingly, it can be understood that the amount charged for the hydrate crystal is likely to decrease even when charged.

Since the anhydrous crystal of 5-chloro-4-(3-chloro-4-methylphenyl)-1H-imidazole-2-carbonitrile has strong electrostatic properties, it may be strongly charged due to friction during transportation or transfer, formulation, and weighing of a dusting powder body, in processes such as production of drug substances and formulation.

When charging occurs, concerns arise over processes, such as a decrease in yield, deterioration of mixing uniformity, and weighing errors caused by the adhesion to piping or packaging materials.

The results of Test Example 5 revealed that making 5-chloro-4-(3-chloro-4-methylphenyl)-1H-imidazole-2-carbonitrile into a hydrate crystal allows the absolute amount charged to be reduced, and that the attenuation speed is considerable even when charged, so the amount charged is quickly lowered. Accordingly, the concerns about processes, quality or the like, caused by the electrification can be alleviated.

### Test Example 6: Antimicrobial Activity Test

Compound under test: Anhydrous crystal, hydrate crystal (Production Example 1)
Strain under test: Staphylococcus pseudintermedius (JCM 17571)

Staphylococcus pseudintermedius that had been cultured on a Luria Broth (LB) agar medium plate (peptone, yeast extract, sodium chloride, agar, from Invitorgen) for 24 hours was suspended in physiological saline to prepare so as to have an absorbance of 0.025 at 600 nm. The compound under test dissolved in DMSO was added thereto so as to have final concentrations of 200, 100, 50, and 25 ppm, and 1 minute later, 10 µL of suspension and 10 mL of physiological saline were added to a syringe and stirred. After that, the mixture was filtered by connecting to a 37 mm quality monitor (manufactured by Pall Corporation) and washed twice with 10 ml of physiological saline. As such, membrane filters were permeated with an LB medium, culturing was performed at 30°C for 72 hours, the presence or absence of the development of colonies was examined to determine a minimum bactericidal concentration (MBC100). The results thereof are shown in Table 6.

**[Table 6]**

| Microbial species | Minimum bactericidal concentration | |
|---|---|---|
| | (MBC100) | |
| | Anhydrous | Hydrate |
| S. pseudintermedius | 50 ppm | 50 ppm |

### Test Example 7: Antifungal Activity Test

Compound under test: Anhydrous crystal, hydrate crystal (Production Example 1)
Strain under test: Microsporum canis (IFM63627)

Microsporum canis was cultured on a 1/10 Sabouraud medium at 32°C for 168 hours to form spores. Then, 10 mL of physiological saline solution (OTSUKA NORMAL SALINE; containing the equivalent of 9 g of sodium chloride in 1000 mL) was directly added over the medium, and the surface was scraped with a disposable loop (Type 1 (1 µL)) to collect a spore suspension containing mycelia. The spore suspension containing mycelia was filtered through a cell strainer (ϕ40 µm). Spores were collected from a filtrate by centrifugation (3500 rpm, 5 minutes), and washed twice with physiological saline to prepare a spore suspension (1 × 107 cells/mL). 10 µL of the spore suspension was inoculated into a Sabouraud medium to which the compound under test dissolved in DMSO was added so as to have a predetermined concentration. Microsporum canis was cultured at 32°C for 72 hours, and then the presence or absence of the development of colonies was examined to determine a minimum inhibitory concentration (MIC100). The results thereof are shown in Table 7.

**[Table 7]**

| Microbial species | Minimum inhibitory concentration (MIC 100) | |
|---|---|---|
| Microsporum canis | Anhydrous | Hydrate |
| | 5 ppm | 5 ppm |

### Test Example 8: Antifungal Activity Test

Compound under test: Anhydrous crystal, hydrate crystal (Production Example 1)
Strain under test: Microsporum canis (IFM63627)

Microsporum canis was cultured on a 1/10 Sabouraud medium at 32°C for 168 hours to form spores. Then, 10 mL of physiological saline solution (OTSUKA NORMAL SALINE; containing the equivalent of 9 g of sodium chloride in 1000 mL) was directly added over the medium, and the surface was scraped with a disposable loop (Type 1 (1 µL)) to collect a spore suspension containing mycelia. The spore suspension containing mycelia was filtered through a cell strainer (ϕ40 µm). Spores were collected from a filtrate by centrifugation (3500 rpm, 5 minutes), and washed twice with physiological saline to prepare a spore suspension (1 × 107 cells/mL). 10 µL of the spore suspension was inoculated into 90 µL of physiological saline solution to which the compound under test dissolved in DMSO was added so as to have a predetermined concentration. Every time a predetermined time lapsed, 10 µL of a test solution containing spores was taken out. Spores were collected from the test solution thus taken out by centrifugation (3500 rpm, 5 minutes), and washed twice with physiological saline to prepare 10 µL of spore suspension. 10 µL of spore suspension was spotted on the Sabouraud medium and cultured at 32°C for 72 hours, and then the presence or absence of the development of colonies was examined to determine a minimum fungicidal concentration (MFC100). The results thereof are shown in Table 8.

**[Table 8]**

| Microbial species | Treatment time | Minimal fungicidal concentration (MFC100) | |
|---|---|---|---|
| | | Anhydrous | Hydrate |
| Microsporum canis | 24 hours | 50 ppm | 50 ppm |
| | 96 hours | 10 ppm | 10 ppm |

### Test Example 9: Antifungal Activity Test

Compound under test: Anhydrous crystal, hydrate crystal (Production Example 1)
Strain under test: Malassezia pachydermatis (IFM56528)

Malassezia pachydermatis that had been cultured on a Sabouraud medium (1% peptone, 4% dextrin, 1.5% agar) for 5 to 7 days was suspended in physiological saline to prepare so as to have an absorbance of 0.025 at 600 nm. The compound under test dissolved in DMSO was added thereto so as to have final concentrations of 200, 100, 50, and 25 ppm. 1 minute later, 10 µL of suspension and 10 mL of physiological saline were added to a syringe and stirred, and then the mixture was filtered and washed by connecting to a 37 mm quality monitor (manufactured by Pall Corporation) and washed. As such, membrane filters were permeated with the Sabouraud medium, culturing was performed at 32°C for 72 hours, the presence or absence of the development of colonies was examined to determine a minimum fungicidal concentration (MFC100). The results thereof are shown in Table 9.

**[Table 9]**

| Microbial species | Minimal fungicidal concentration (MFC100) | |
|---|---|---|
| | Anhydrous | Hydrate |
| Malassezia pachydermatis | 50 ppm | 50 ppm |

### Test Example 10: Antifungal Activity Test

Compound under test: Anhydrous crystal, hydrate crystal (Production Example 1)
Strain under test: Malassezia furfur (IFM 55951), Malassezia sympodialis (IFM 48588), and Malassezia globosa (IFM 51946)

Fungi of Malassezia (Malassezia furfur, Malassezia sympodialis, and Malassezia globosa) that had been cultured on a CHROMagar (trademark) MalassezialCandida raw medium (peptone, specialized enzyme-substrate mixture, chloramphenicol, olive oil, agar, manufactured by KANTO CHEMICAL CO., INC.) for 5 to 7 days was suspended in physiological saline to prepare so as to have an absorbance of 0.025 at 600 nm. The compound under test dissolved in DMSO was added thereto so as to have final concentrations of 200, 100, 50, and 25 ppm. 1 minute later, 10 µL of suspension and 10 mL of physiological saline were added to a syringe and stirred, and then the mixture was filtered by connecting to a 37 mm quality monitor (manufactured by Pall Corporation) and washed twice with 10 ml of physiological saline. The membrane filters were removed from the quality monitor, and placed and cultured on the CHROMagar (trademark) MalassezialCandida raw medium at 32°C for 72 hours, and the presence or absence of the development of colonies was examined to determine a minimum fungicidal concentration (MFC100). The results thereof are shown in Table 10.

**[Table 10]**

| Microbial species | Minimal fungicidal concentration (MFC100) | |
|---|---|---|
| | Anhydrous | Hydrate |
| Malassezia furfur | 50 ppm | 50 ppm |
| Malassezia sympodialis | 50 ppm | 50 ppm |
| Malassezia globosa | 50 ppm | 50 ppm |

### Test Example 11: Antifungal Activity Test

Compound under test: Anhydrous crystal, hydrate crystal (Production Example 1)
Strain under test: Malassezia restricta (IFM55992)

A fungus of Malassezia (Malassezia restricta) that had been cultured on a CHROMagar (trademark) MalassezialCandida raw medium (peptone, specialized enzyme-substrate mixture, chloramphenicol, olive oil, agar, manufactured by KANTO CHEMICAL CO., INC.) for 5 to 7 days was suspended in physiological saline to prepare so as to have an absorbance of 0.025 at 600 nm. The compound under test dissolved in DMSO was added thereto so as to have final concentrations of 200, 100, 50, and 25 ppm. 1 minute later, 10 µL of suspension and 10 mL of physiological saline were added to a syringe and stirred, and then the mixture was filtered by connecting to a 37 mm quality monitor (manufactured by Pall Corporation) and washed twice with 10 ml of physiological saline. The membrane filters were removed from the quality monitor, and placed and cultured on a Leeming & Notman agar Modified medium (MLNA medium) (peptone, glucose, yeast extract, dried bovine bile, glycerol, glycerin stearate, Tween 60, olive oil, agar) at 32°C for 72 hours, and the presence or absence of the development of colonies was examined to determine a minimum fungicidal concentration MFC100). The results thereof are shown in Table 11.

**[Table 11]**

| Microbial species | Minimal fungicidal concentration (MFC100) | |
|---|---|---|
| | Anhydrous | Hydrate |
| Malassezia restricta | 200 ppm | 200 ppm |

### Test Example 12: Antifungal Activity Test

Compound under test: Anhydrous crystal, hydrate crystal (Production Example 1)
Strain under test: Trichophyton rubrum (ATCCMYA4607)

Trichophyton rubrum was cultured on a Sabouraud medium at 32°C for 168 hours to form spores. Mycelia were peeled from a medium surface and collected into a tube, physiological saline solution (OTSUKANORMAL SALINE; containing the equivalent of 9 g of sodium chloride in 1000 mL) was added thereto, and the entire mixture was mixed and dispersed with a disposable loop (Type 1 (1 µL)) to collect a spore suspension containing mycelia. The spore suspension containing mycelia was filtered through a cell strainer (ϕ40 µm). Spores were collected from a filtrate by centrifugation (3500 rpm, 5 minutes), and washed twice with physiological saline to prepare a spore suspension (1 × 106 cells/mL). 10 µL of the spore suspension was inoculated into the Sabouraud medium to which the test compound dissolved in DMSO was added so as to have a predetermined concentration. Trichophyton rubrum was cultured at 32°C for 72 hours, and then the presence or absence of the development of colonies was examined to determine a minimum inhibitory concentration (MIC100). The results thereof are shown in Table 12.

**[Table 12]**

| Microbial species | Minimum inhibitory concentration (MIC 100) | |
|---|---|---|
| | Anhydrous | Hydrate |
| Trichophyton rubrum | 5 ppm | 5 ppm |

### Test Example 13: Antifungal Activity Test

Compound under test: Anhydrous crystal, hydrate crystal (Production Example 1)
Strain under test: Trichophyton rubrum (ATCCMYA4607)

Trichophyton rubrum was cultured on a Sabouraud medium at 32°C for 168 hours to form spores. Mycelia were peeled from a medium surface and collected into a tube, physiological saline solution (OTSUKANORMAL SALINE; containing the equivalent of 9 g of sodium chloride in 1000 mL) was added thereto, and the entire mixture was mixed and dispersed with a disposable loop (Type 1 (1 µL)) to collect a spore suspension containing mycelia. The spore suspension containing mycelia was filtered through a cell strainer (ϕ40 µm). Spores were collected from a filtrate by centrifugation (3500 rpm, 5 minutes), and washed twice with physiological saline to prepare a spore suspension (1 × 107 cells/mL). 10 µL of the spore suspension was inoculated into 90 µL of physiological saline solution to which the test compound dissolved in DMSO was added so as to have a predetermined concentration. Every time a predetermined time lapsed, 10 µL of a test solution containing spores was taken out. Spores were collected from the test solution thus taken out by centrifugation (3500 rpm, 5 minutes), and washed twice with physiological saline to prepare 10 µL of spore suspension. 10 µL of spore suspension was spotted on the Sabouraud medium and cultured at 32°C for 72 hours, and then the presence or absence of the development of colonies was examined to determine a minimum fungicidal concentration (MFC100). The results thereof are shown in Table 13.

**[Table 13]**

| Microbial species | Treatment time | Minimal fungicidal concentration (MFC 100) | |
|---|---|---|---|
| | | Anhydrous | Hydrate |
| Trichophyton rubrum | 1 hour | 50 ppm | 50 ppm |
| | 48 hours | 10 ppm | 10 ppm |
| | 72 hours | 5 ppm | 5 ppm |

### Test Example 14: Antimicrobial Activity Test

A minimum bactericidal concentration (MBC100) for Staphylococcus pseudintermedius (JCM 17571) was determined in the same method as in Test Example 6 except that the hydrate crystal (Production Example 2) was used as the compound under test. The results thereof are shown in Table 14.

**[Table 14]**

| Microbial species | Minimum bactericidal concentration (MBC100) |
|---|---|
| | Hydrate (Production Example 2) |
| S. pseudintermedius | 50 ppm |

### Test Example 15: Antifungal Activity Test

A minimum inhibitory concentration (MIC100) for Microsporum canis (IFM63627) was determined in the same method as in Test Example 7 except that the hydrate crystal (Production Example 2) was used as the compound under test. The results thereof are shown in Table 15.

**[Table 15]**

| Microbial species | Minimum inhibitory concentration (MIC 100) |
|---|---|
| | Hydrate (Production Example 2) |
| Microsporum canis | 5 ppm |

### Test Example 16: Antifungal Activity Test

A minimum fungicidal concentration (MFC100) for Microsporum canis (IMF63627) was determined in the same method as in Test Example 8 except that the hydrate crystal (Production Example 2) was used as the compound under test. The results thereof are shown in Table 16.

**[Table 16]**

| Microbial species | Treatment time | Minimal fungicidal concentration (MFC 100) |
|---|---|---|
| | | Hydrate (Production Example 2) |
| Microsporum canis | 24 hours | 50 ppm |
| | 96 hours | 10 ppm |

### Test Example 17: Antifungal Activity Test

A minimum fungicidal concentration (MFC100) for Malassezia pachydermatis (IFM56528) was determined in the same method as in Test Example 9 except that the hydrate crystal (Production Example 2) was used as the compound under test. The results thereof are shown in Table 17.

**[Table 17]**

| Microbial species | Minimal fungicidal concentration (MFC 100) |
|---|---|
| | Hydrate (Production Example 2) |
| Malassezia pachydermatis | 50 ppm |

### Test Example 18: Antifungal Activity Test

Minimum fungicidal concentrations (MFC100) for Malassezia furfur (IFM 55951), Malassezia sympodialis (IFM 48588), and Malassezia globosa (IFM 51946) were determined in the same method as in Test Example 10 except that the hydrate crystal (Production Example 2) was used as the compound under test. The results thereof are shown in Table 18.

**[Table 18]**

| Microbial species | Minimal fungicidal concentration (MFC 100) |
|---|---|
| | Hydrate (Production Example 2) |
| Malassezia furfur | 50 ppm |
| Malassezia sympodialis | 50 ppm |
| Malassezia globosa | 50 ppm |

### Test Example 19: Antifungal Activity Test

A minimum fungicidal concentration (MFC100) for Malassezia restricta (IFM55992) was determined in the same method as in Test Example 11 except that the hydrate crystal (Production Example 2) was used as the compound under test. The results thereof are shown in Table 19.

**[Table 19]**

| Microbial species | Minimal fungicidal concentration (MFC 100) |
|---|---|
| | Hydrate (Production Example 2) |
| Malassezia restricta | 200 ppm |

### Test Example 20: Antifungal Activity Test

A minimum inhibitory concentration (MIC100) for Trichophyton rubrum (ATCCMYA4607) was determined in the same method as in Test Example 13 except that the hydrate crystal (Production Example 2) was used as the compound under test. The results thereof are shown in Table 20.

**[Table 20]**

| Microbial species | Treatment time | Minimal fungicidal concentration (MFC 100) |
|---|---|---|
| | | Hydrate (Production Example 2) |
| Trichophyton rubrum | 1 hour | 50 ppm |
| | 48 hours | 10 ppm |
| | 72 hours | 5 ppm |

### Test Example 21: Antifungal Activity Test

A minimum inhibitory concentration (MIC100) for Trichophyton rubrum (ATCCMYA4607) was determined in the same method as in Test Example 12 except that the hydrate crystal (Production Example 2) was used as the compound under test. The results thereof are shown in Table 21.

**[Table 21]**

| Microbial species | Minimal fungicidal concentration (MFC 100) |
|---|---|
| | Hydrate (Production Example 2) |
| Trichophyton rubrum | 5 ppm |

## Claims

1. A hydrate crystal of 5-chloro-4-(3-chloro-4-methylphenyl)-1H-imidazole-2-carbonitrile.

2. The hydrate crystal according to claim 1, wherein the hydrate crystal is a monohydrate crystal.

3. The hydrate crystal according to claim 1, wherein a powder X-ray diffraction pattern measured using a Cu-Kα radiation source has at least one peak at a diffraction angle (2θ ± 0.2°) of 9.8, 12.8, 14.6, 16.0, 19.5, 24.2, 25.3, 25.7, 26.7, 27.7, 28.5, 28.8, 29.5, 30.3, 32.3, 33.1, 34.1, 35.7, 36.4, 37.4, 39.5, 40.1, 43.3, or 44.3.

4. The hydrate crystal according to claim 1, wherein a powder X-ray diffraction pattern measured using the Cu-Kα radiation source has at least one peak at a diffraction angle (2θ ± 0.2°) of 9.8, 12.8, 14.6, 16.0, 19.5, 25.3, 25.7, 26.7, or 29.5.

5. The hydrate crystal according to claim 3 or 4, wherein the at least one peak is at least the top 5 peaks with highest intensity.

6. The hydrate crystal according to claim 1, wherein at least one endothermic peak falls within a range selected from 93 to 100°C and 135 to 145°C in thermogravimetric-differential thermal analysis (TG-DTA) or differential scanning calorimetry analysis (DSC).

7. The hydrate crystal according to claim 1, wherein an infrared absorption spectrum measured by a potassium bromide disk method has at least one peak at a position (cm-1) selected from 835 ±2, 860 ±2, 1014 ± 2, 1053 ±2, 1157 ± 2, 1315 ± 2, 1427 ± 2, 1454 ± 2, 1500 ± 2, 1581 ± 2, 1654 ±2 and 2245 ±2.

8. The hydrate crystal according to claim 1, wherein a Raman spectroscopy spectrum has at least one peak at a position (cm-1) selected from 1021 ± 2, 1152 ± 2, 1240 ± 2, 1281 ± 2, 1379 ± 2, 1409 ± 2, 1424 ± 2, 1451 ±2, 1507 ± 2, 1573 ±2, 1607 ± 2 and 2241 ± 2.

9. The hydrate crystal according to claim 1, wherein a moisture content measured by a Karl Fischer method (volumetric titration method) is 6.5 to 6.8% by mass.

10. A preparation comprising the hydrate crystal according to claim 1 or 2 and a pharmacologically acceptable additional ingredient.

11. The preparation according to claim 10, wherein the preparation is a pharmaceutical, a quasi-drug, a veterinary drug, or a cosmetic.

12. The preparation according to claim 10, wherein the preparation is an antimicrobial agent.

13. The preparation according to claim 10, wherein the preparation is an antifungal agent or an antibacterial agent.

14. The preparation according to claim 10, wherein the preparation is for ameliorating a symptom or a disease caused by a fungus or bacterium.

15. The preparation according to claim 14, wherein the symptom or the disease is a skin disease.

16. The preparation according to claim 12 or 13, for use against at least one fungus selected from a genus Candida, a genus Malassezia, a genus Trichophyton, a genus Microsporum, a genus Arthroderma, a genus Aspergillus and a genus Cryptococcus.

17. The preparation according to claim 12 or 13, for use against at least one bacterium selected from a Staphylococcus, a genus Streptococcus, a genus Pasteurella, a genus Escherichia, a genus Pseudomonas, a genus Proteus, and a genus Klebsiella.

18. Use of a hydrate crystal of 5-chloro-4-(3-chloro-4-methylphenyl)-1H-imidazole-2-carbonitrile, in production of a pharmaceutical, a quasi-drug, a veterinary drug or a cosmetic.

19. Use of a hydrate crystal of 5-chloro-4-(3-chloro-4-methylphenyl)-1H-imidazole-2-carbonitrile, in production of an antimicrobial agent.

20. A method for ameliorating a symptom or a disease caused by a fungus or a bacterium, comprising
administering to a subject in need thereof, an effective amount of a hydrate crystal of 5-chloro-4-(3-chloro-4-methylphenyl)-1H-imidazole-2-carbonitrile.
